(19) Europäisches Patentamt
European Patent Office
Office européen des brevets

(11) Veröffentlichungsnummer : **0 113 091**
**B1**

(12) # EUROPÄISCHE PATENTSCHRIFT

(45) Veröffentlichungstag der Patentschrift : **30.07.86**

(51) Int. Cl.[4] : **A 01 N 43/90**, A 01 N 43/42

(21) Anmeldenummer : **83112720.4**

(22) Anmeldetag : **17.12.83**

(54) **Mikrobizide Mittel auf Chinoloncarbonsäure-Basis.**

(30) Priorität : **29.12.82 DE 3248507**

(43) Veröffentlichungstag der Anmeldung : **11.07.84 Patentblatt 84/28**

(45) Bekanntmachung des Hinweises auf die Patenterteilung : **30.07.86 Patentblatt 86/31**

(84) Benannte Vertragsstaaten : **AT BE CH DE FR GB IT LI NL**

(56) Entgegenhaltungen :
**EP-A- 0 000 203**
**DE-A- 3 033 157**
**DE-B- 2 804 097**
**DE-C- 3 007 006**
**US-A- 4 146 625**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Grohe, Klaus, Dr.**
**Am Wasserturm 10**
**D-5068 Odenthal (DE)**
Erfinder : **Petersen, Uwe, Dr.**
**Auf dem Forst 4**
**D-5090 Leverkusen 1 (DE)**
Erfinder : **Kuck, Karl-Heinz, Dr.**
**Heerstrasse 24**
**D-4018 Langenfeld (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft die Verwendung von teilweise bekannten 1-Cyclopropyl-1,4-dihydro-4-oxo-3-chinolincarbonsäuren als Mikrobizide im Pflanzenschutz.

Es sind bereits bestimmte 1-Cyclopropyl-1,4-dihydro-4-oxo-3-chinolincarbonsäuren, wie z. B. die 1-Cyclopropyl-6-fluor-1,4-dihydro-4-oxo-7-(1-piperazinyl)-3-chinolincarbonsaüre, bekannt (vgl. EP 49 355). Über eine mikrobizide Wirkung auf dem Pflanzenschutzgebiet ist nichts bekannt, nur ihre Verwendung auf dem Pharmagebiet.

Weiterhin ist bekannt geworden, daß bestimmte Chinoloncarbonsäurederivate eine pflanzenbakterizide Wirksamkeit besitzen. So zeigt die 7-Chlor-1-ethyl-6-fluor-1,4-dihydro-4-oxo-3-chinolincarbonsäure eine bakterizide Wirkung speziell gegen Erwinia amylovora (Feuerbrand an Obstbäumen) (vgl. Europäische Patentanmeldung 0203). Die Wirksamkeit ist jedoch bei niedrigen Anwendungskonzentrationen nicht immer befriedigend.

Es wurde gefunden, daß die teilweise bekannten 1-Cyclopropyl-1,4-dihydro-4-oxo-3-chinolincarbonsäuren der Formel (I)

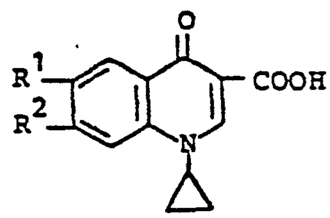

in welcher

$R^1$ für Wasserstoff, Fluor, Chlor, Brom oder Nitro steht,

$R^2$ für Wasserstoff, Chlor, Fluor oder für die Gruppe

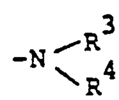

steht, wobei

$R^3$ und $R^4$ gleich oder verschieden sind und für gegebenenfalls durch Hydroxy substituiertes Alkyl mit 1 bis 4 Kohlenstoffatomen stehen oder $R^3$ und $R^4$ gemeinsam mit dem Stickstoffatom, an dem sie stehen, einen 5- oder 6-gliedrigen, gesättigten oder partiell ungesättigten, gegebenenenfalls ein- oder mehrfach, gleich oder verschieden substituierten Heterocyclus bilden, der gegebenenfalls noch Sauerstoff, Schwefel, eine SO-, $SO_2$- oder $NR^5$-Gruppe enthalten kann, wobei als Substituenten der Heterocyclen Alkyl mit 1 bis 6 Kohlenstoffatomen, Alkenyl mit 2 bis 6 Kohlenstoffatomen, Phenyl oder in Hydroxy in Betracht kommen, und

$R^5$ für Wasserstoff, für gegebenenfalls ein- oder mehrfach, gleich oder verschieden durch Hydroxy oder Alkoxy mit 1 bis 4 Kohlenstoffatomen substituiertes Alkyl mit 1 bis 12 Kohlenstoffatomen, für gegebenenfalls durch Nitro, Amino oder die Gruppe $—O—CH_2—O—$ substituiertes Phenylalkyl mit 1 bis 3 Kohlenstoffatomen im Alkylteil, für gegebenenfalls ein- oder mehrfach, gleich oder verschieden durch Halogen, Halogenalkyl mit 1 oder 2 Kohlenstoffatomen und mit bis zu 5 Halogenatomen, Hydroxy, Alkoxy mit 1 bis 3 Kohlenstoffatomen oder durch die Gruppe $—O—CH_2—O—$ substituiertes Phenyl, für Heteroaryl oder für die Gruppierung

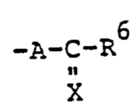

steht, wobei

A für eine Alkylenkette mit 1 bis 4 Kohlenstoffatomen steht,

$R^6$ für Wasserstoff, für Alkyl mit 1 bis 6 Kohlenstoffatomen oder für gegebenenfalls ein- oder mehrfach durch Hydroxy, Halogen oder Alkoxy substituiertes Phenyl steht und

X für Sauerstoff oder die Gruppierungen =NOR', =N—NH—R'' oder $(OR''')_2$ steht, wobei

R' für Wasserstoff, Alkyl mit 1 bis 6 Kohlenstoffatomen, Cycloalkyl mit 5 oder 6 Kohlenstoffatomen, Benzyl, Chlorbenzyl oder Tetrahydropyranyl steht,

R'' für Methyl, Phenyl, Carbamoyl oder Thiocarbamoyl und

R''' für Methyl, Ethyl oder $(OR''')_2$ für

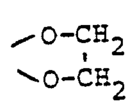

stehen, und deren pflanzenverträgliche Säureadditions-, Alkali-, Erdalkali- oder Schwermetallsalze und gegebenenfalls die Hydrate starke mikrobizide Eigenschaften auf dem Pflanzenschutzgebiet aufweisen.

Überraschenderweise zeigen die 1-Cyclopropyl-1,4-dihydro-4-oxo-3-chinolincarbonsäuren der Formel I eine wesentlich breitere und bessere Wirkung vor allem gegen pflanzenpathogene Bakterien als die aus dem Stand der Technik bekannten vergleichbaren Chinoloncarbonsäuren, wie beispielsweise die 7-Chlor-1,4-dihydro-1-ethyl-6-fluor-4-oxo-3-chinolincarbonsäure, welche wirkungsmäßig die chemisch naheliegendsten Verbindungen sind.

Die neue Verwendung der Wirkstoffe stellt somit eine Bereicherung der Technik dar.

Die erfindungsgemäß verwendbaren Verbindungen sind durch die Formel (I) definiert. In dieser Formel stehen vorzugsweise

$R^1$ für Wasserstoff, Fluor, Chlor oder für Nitro,

$R^2$ für Chlor, Fluor oder für die Gruppe

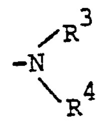 ,

wobei

$R^3$ und $R^4$ gleich oder verschieden sind und für Alkyl mit 1 bis 3 Kohlenstoffatomen, für einfach durch Hydroxy substituiertes Alkyl mit 2 oder 3 Kohlenstoffatomen oder $R^3$ und $R^4$ gemeinsam mit dem Stickstoffatom, an dem sie stehen, für gegebenenfalls ein- bis dreifach durch Alkyl mit 1 bis 4 Kohlenstoffatomen, Alkenyl mit 2 bis 4 Kohlenstoffatomen, Phenyl oder durch Hydroxy substituiertes 1-Pyrrolidinyl, 1-Piperidinyl, 1,2,3,6-Tetrahydro-1-pyridyl, 4-Morpholinyl, 4-Thiomorpholinyl, 1,1-Dioxo-4-thiomorpholinyl stehen oder $R^3$ und $R^4$ gemeinsam mit dem Stickstoffatom, an dem sie stehen, den Heterocyclus

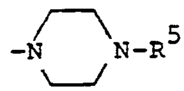

bilden, der wie oben angegeben, substituiert sein kann und

$R^5$ für Wasserstoff, für Alkyl mit 1 bis 12 Kohlenstoffatomen, für einfach durch Hydroxy substituiertes Alkyl mit 1 bis 4 Kohlenstoffatomen, für einfach durch Methoxy substituiertes Alkyl mit 1 bis 4 Kohlenstoffatomen, für gegebenenfalls ein- bis dreifach durch Hydroxy, Alkoxy mit 1 oder 2 Kohlenstoffatomen, Trifluormethyl, Fluor, Dioxymethylen substituiertes Phenyl, für Pyridyl, für Pyrimidinyl, für gegebenenfalls durch Dioxymethylen substituiertes Phenalkyl mit 1 oder 2 Kohlenstoffatomen im Alkylteil oder für die Gruppierung

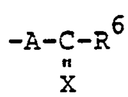 ,

wobei

A für eine Alkylenkette mit 1 bis 3 Kohlenstoffatomen,

$R^6$ für Wasserstoff, für Alkyl mit 1 bis 4 Kohlenstoffatomen, für gegebenenfalls ein- bis dreifach, gleich oder verschieden durch Hydroxy, Fluor, Chlor oder Methoxy substituiertes Phenyl und

X für Sauerstoff oder die Gruppierungen $=NOR'$, $=N—NHR''$ oder $(OR''')_2$, wobei

R' für Alkyl mit 1 bis 3 Kohlenstoffatomen, für Benzyl oder für Tetrahydropyranyl,

R'' für Methyl, Carbamoyl oder Thiocarbamoyl und

R''' für Methyl, Ethyl oder $(OR''')_2$ für

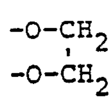

und deren pflanzenverträgliche Säureadditions-, Alkali-, Erdalkali- oder Schwermetallsalze und die Hydrate.

Als Beispiele seien außer den in den Herstellungsbeispielen gennanten Verbindungen die folgenden genannt :

1-Cyclopropyl-6,7-difluor-1,4-dihydro-4-oxo-3-chinolincarbonsäure,
1-Cyclopropyl-6-chlor-1,4-dihydro-4-oxo-7-(4-β-hydroxy-ethyl-1-piperazinyl)-3-chinolincarbonsäure,
1-Cyclopropyl-6-chlor-1,4-dihydro-4-oxo-7-(4-morpholinyl)-3-chinolincarbonsäure,
1-Cyclopropyl-6-chlor-1,4-dihydro-4-oxo-7-(4-methyl-1-piperazinyl)-3-chinolincarbonsäure,
1-Cyclopropyl-6-fluor-1,4-dihydro-4-oxo-7-(2,5-dimethyl-1-piperazinyl)-3-chinolincarbonsäure,
1-Cyclopropyl-6-fluor-1,4-dihydro-4-oxo-7-(2,4,5-trimethyl-1-piperazinyl)-3-chinolincarbonsäure,
1-Cyclopropyl-6-fluor-1,4-dihydro-4-oxo-7-(3,4,5-trimethyl-1-piperazinyl)-3-chinolincarbonsäure,

1-Cyclopropyl-6-fluor-1,4-dihydro-4-oxo-7-(4-ethyl-3,5-dimethyl-1-piperazinyl)-3-chinolincarbonsäure,
1-Cyclopropyl-6-fluor-1,4-dihydro-4-oxo-7-(3,4-di-ethyl-1-piperazinyl)-3-chinolincarbonsäure,
1-Cyclopropyl-6-fluor-1,4-dihydro-4-oxo-7-(3-n-propyl-1-piperazinyl)-3-chinolincarbonsäure,
1-Cyclopropyl-6-fluor-1,4-dihydro-4-oxo-7-(3-isopropyl-1-piperazinyl)-3-chinolincarbonsäure,
1-Cyclopropyl-6-fluor-1,4-dihydro-4-oxo-7-(4-sek.-butyl-3-methyl-1-piperazinyl)-3-chinolincarbonsäure,
1-Cyclopropyl-6-chlor-1,4-dihydro-4-oxo-7-(4-tert.-butyl-3-methyl-1-piperazinyl)-3-chinolincarbonsäure
und gegebenenfalls deren pflanzenverträgliche Säureadditions-, Alkali-, Erdalkali- oder Schwermetallsalze und Hydrate.

Die erhaltenen erfindungsgemäßen Verbindungen der Formel I können gegebenenfalls mit einer organischen oder anorganischen Säure in ein Salz übergeführt werden. Zur Salzbildung geeignete Säuren sind z. B. Halogenwasserstoffsäuren wie Salzsäure, Bromwasserstoffsäure, lodwasserstoffsäure, Schwefelsäure, Essigsäure, Zitronensäure, Ascorbinsäure, Methansulfonsäure und Benzolsulfonsäure. Als Alkali- bzw. Erdalkalisalze sind vorzugsweise Natrium-, Kalium-, Calcium- und Magnesiumsalze, als Schwermetallsalze vorzugsweise Kupfer-, Zink- und Mangansalze geeignet.

Die Salze der Verbindungen der Formel (I) können in einfacher Weise nach üblichen Salzbildungsmethoden, z. B. durch Lösen einer Verbindung in einem geeigneten inerten Lösungsmittel und Hinzufügen einer der oben genannten Saüren, erhalten werden und in bekannter Weise, z. B. Abfiltrieren, isoliert und gegebenenfalls durch Waschen mit einem inerten organischen Lösungsmittel gereinigt werden.

Einzelne der erfindungsgemäßen Verbindungen sind neu, sie können jedoch nach prinzipiell bekannten Verfahren hergestellt werden. Vergleiche dazu die Herstellungsbeispiele.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So werden z. B. bakterizide Mittel im Pflanzenschutz zur Bekämpfung von Pseudomonadaceae, Rhizobiaceae, Enterobacteriaceae, Corynebacteriaceae und Streptomycetaceae eingesetzt.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Insbesondere sind die erfindungsgemäßen Verbindungen wirksam gegen Bakterien der Gattungen Xanthomonas, z. B. gegen Xanthomonas oryzae, Pseudomonas, z. B. gegen Pseudomonas lachrymans und Erwinia, z. B. gegen Erwinia carotovora var. atroseptica.

Einige der Verbindungen sind bei Anwendung entsprechender Konzentrationen auch fungizid wirksam. Besonders erwähnt seien die Wirkungen gegen Oomyceten, gegen Schorfpilze (Venturia) und die protektive und systemische Wirkung gegen Pyricularia oryzae.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise kergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischem Mehlen sowie Granulate aus organischen Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Staüben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 % am Wirkungsort erforderlich.

In den nachfolgenden Beispielen wird die im folgenden angegebene bekannte Verbindung als Vergleichssubstanz eingesetzt.

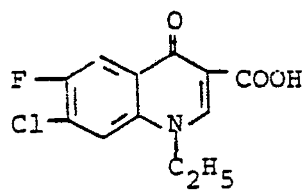

(A)

7-Chlor-1-ethyl-6-fluor-1,4-dihydro-4-oxo-3-chinolincarbonsäure.

Beispiel A

Xanthomonas oryzae-Test/Bakteriose/Reis
protektiv

Lösungsmittel : 48,5 Gewichtsteile Dimethylformamid
Emulgator : 1,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach 48 Stunden werden die Pflanzen mit einer wäßrigen Suspension von Xanthomonas oryzae durch Stichverletzung der Blätter inokuliert. Nach einer 48-stündigen Inkubation bei 100 % rel. Luftfeuchtigkeit verbleiben die Pflanzen 10 Tage bis zur Auswertung in einem Gewächshaus bei 24 bis 26°C und 70 bis 80 % rel. Luftfeuchtigkeit.

Eine deutliche Überlegenheit in der Wirksamkeit gegenüber dem Stand der Technik zeigen bei diesem Test die Verbindungen gemäß folgender Herstellungsbeispiele :

(Siehe Tabelle A Seite 6 ff.)

Tabelle A

Xanthomonas oryzae-Test/Bakteriose/Reis
protektiv

| Wirkstoffe | Wirkstoff-konzentration in % | Krankheitsbefall in % der unbehandelten Kontrolle |
|---|---|---|
| 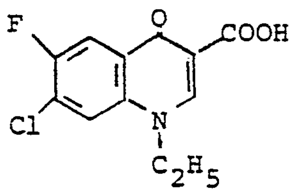 (bekannt) | 0,025 | 75 |
| 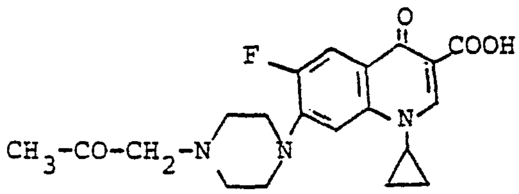 | 0,025 | 20 |
| 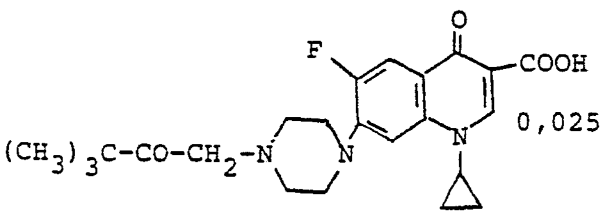 | 0,025 | 37,5 |
| 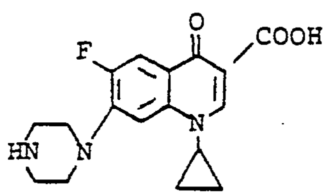 x HCl | 0,025 | 20 |
| 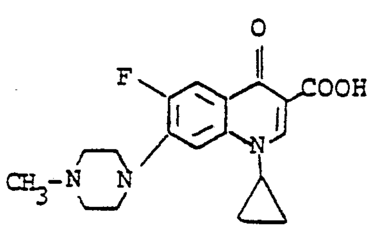 | 0,025 | 25 |
| 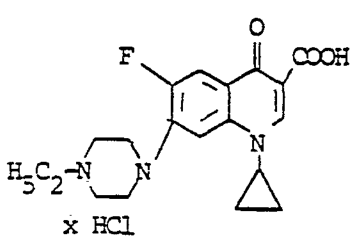 x HCl | 0,025 | 25 |

Tabelle A (Fortsetzung)

Xanthomonas oryzae-Test/Bakteriose/Reis
protektiv

| Wirkstoffe | Wirkstoff-konzentration in % | Krankheitsbefall in % der unbehandelten Kontrolle |
|---|---|---|
| 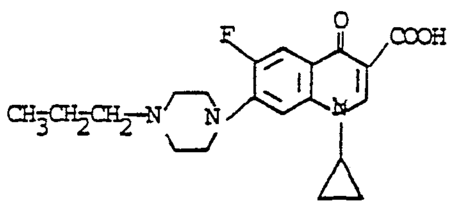 | 0,025 | 25 |
| 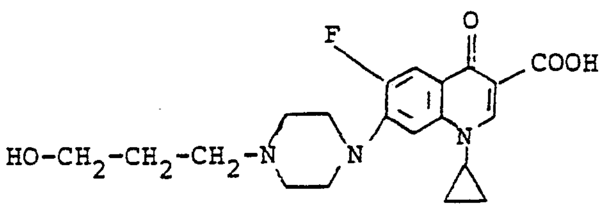 | 0,025 | 25 |
| 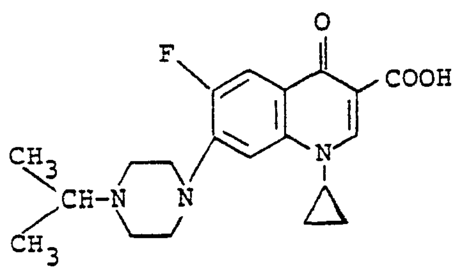 | 0,025 | 25 |

Beispiel B

Xanthomonas oryzae-Test/Bakteriose/Reis
systemisch

Lösungsmittel : 48,5 Gewichtsteile Dimethylformamid
Emulgator : 1,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften werden 100 ml der Wirkstoffzubereitung auf Einheitserde gegossen, in der junge Pflanzen angezogen wurden. 3 Tage nach der Behandlung werden die Pflanzen mit einer wäßrigen Suspension von Xanthomonas oryzae durch Stichverletzung inokuliert. Danach verbleiben die Pflanzen 14 Tage bis zur Auswertung in einem Gewächshaus bei 24 bis 26 °C und 70 bis 80 % rel. Luftfeuchtigkeit.

Eine deutliche Überlegenheit in der Wirksamkeit gegenüber dem Stand der Technik zeigen bei diesem Test z. B. die Verbindungen gemäß folgender Herstellungsbeispiele :

Tabelle B

Xanthomonas oryzae-Test/Bakteriose/Reis systemisch

| Wirkstoffe | Aufwandmenge in mg Wirkstoff pro 100 $cm^2$ | Krankheitsbefall in % der unbehandelten Kontrolle |
|---|---|---|
| 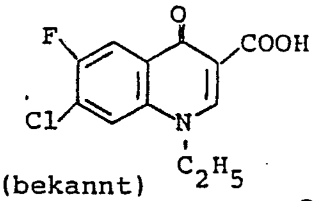 (bekannt) | 10 | 25 |
| 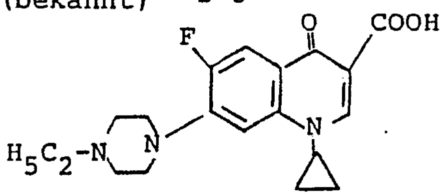 | 10 | 6 |
| 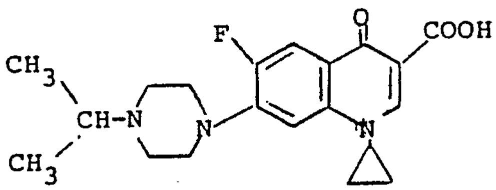 | 10 | 12 |
| 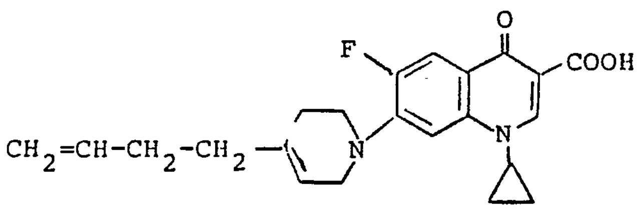 | 10 | 12 |

Beispiel C

Agarplattentest

Verwendeter Nährboden

15 Gewichtsteile Agar-Agar
10 Gewichtsteile Saccharose
8 Gewichtsteile Caseinhydrolysat
4 Gewichtsteile Hefeextrakt
2 Gewichtsteile Dikaliumhydrogenphosphat
0,3 Gewichtsteile Magnesiumphosphat

werden in 1 000 ml destilliertem Wasser gelöst und 30 Minuten bei 121 °C autoklaviert.

Lösungsmittel : 10 Gewichtsteile Dimethylformamid Mengenverhältnis von Lösungsmittel zu Nährboden : 0,2 : 99,8

Zur Herstellung einer zweckmäßigen Wirkstoffzubereitung vermischt man 1 Gewichtsteil Wirkstoff mit der angegebenen Menge Lösungsmittel.

Das Konzentrat wird im genannten Mengenverhältnis mit dem flüssigen Nährboden gründlich vermischt und in Petrischalen gegossen.

Ist der Nährboden erkaltet und fest, werden die Platten mit Mikroorganismen beimpft und bei ca. 21 °C inkubiert :

Die Auswertung erfolgt nach 2 Tagen, wobei die Wachstumshemmung als Maß für die Wirkung der Präparate herangezogen wird.

Eine deutliche Überlegenheit gegenüber dem Stand der Technik zeigen in diesem Test die Verbindungen gemäß folgender Herstellungsbeispiele :

Tabelle C

Agarplatten-Test

| Wirkstoffe | Wirkstoffkonzentration ppm | Wachstum in Wertzahlen (1=kein Wachstum, 9=Kontrolle)<br>Mikroorganismen<br>Agrobacterium tumefaciens | Corynebacterium michiganense | Erwinia carotovora var. atroseptica | Erwinia mangiferae |
|---|---|---|---|---|---|
| 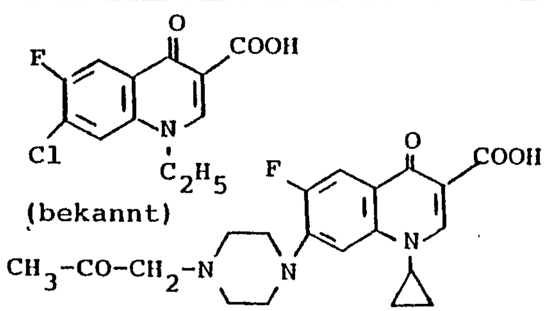<br>(bekannt) | 50 | 5 | 5 | 2 | 7 |
| | 50 | 2 | 1 | 1 | 1 |
| 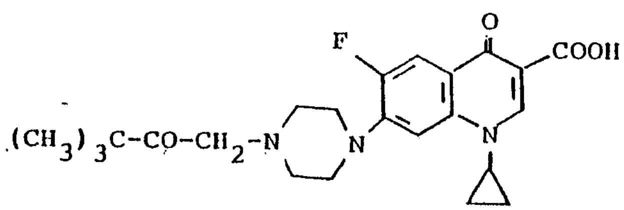 | 50 | 2 | 1 | 1 | 1 |

Tabelle C (Fortsetzung)

Agarplatten-Test

| Wirkstoffe | Wirkstoffkonzentration ppm | Wachstum in Wertzahlen (1=kein Wachstum, 9=Kontrolle) Mikroorganismen: Agrobacterium tumefaciens | Corynebacterium michiganense | Erwinia carotovora var. atroseptica | Erwinia mangiferae |
|---|---|---|---|---|---|
| 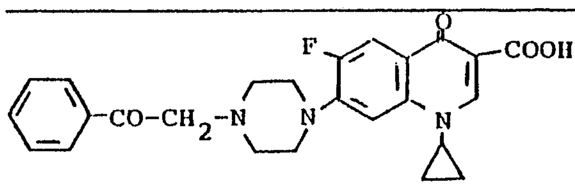 | 50 | 2 | 1 | 1 | 1 |
| 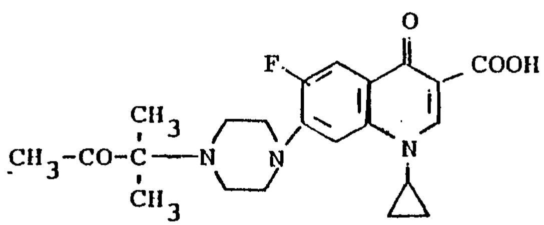 | 50 | 2 | 1 | 1 | 1 |

Tabelle C (Fortsetzung)

Agarplatten-Test

| Wirkstoffe | Wirkstoffkonzentration ppm | Wachstum in Wertzahlen (1=kein Wachstum, 9=Kontrolle) Mikroorganismen: Agrobacterium tumefaciens | Corynebacterium michiganense | Erwinia carotovora var. atroseptica | Erwinia mangiferae |
|---|---|---|---|---|---|
| 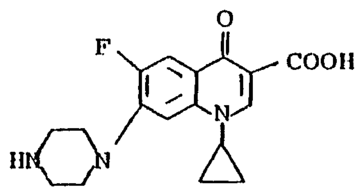 x HCl | 50 | 2 | 2 | 1 | 1 |
| 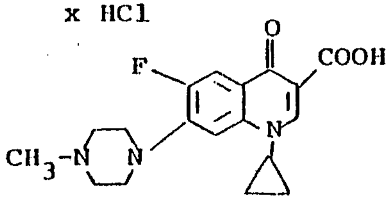 | 50 | 1 | 1 | 1 | 1 |
| 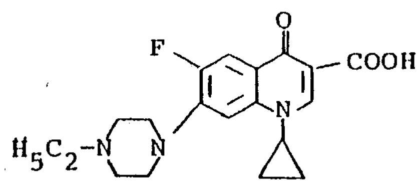 | 50 | 2 | 2 | 1 | 2 |

Tabelle C (Fortsetzung)

Agarplatten-Test

| Wirkstoffe | Wirkstoffkonzentration ppm | Wachstum in Wertzahlen (1=kein Wachstum, 9=Kontrolle) Mikroorganismen: Agrobacterium tumefaciens | Corynebacterium michiganense | Erwinia carotovora var. atroseptica | Erwinia mangiferae |
|---|---|---|---|---|---|
| 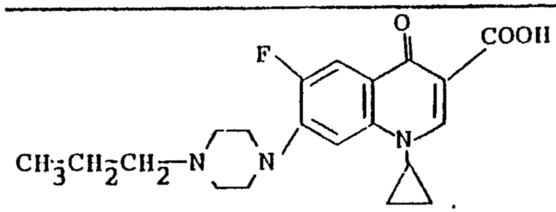 | 50 | 3 | 2 | 1 | 2 |
| 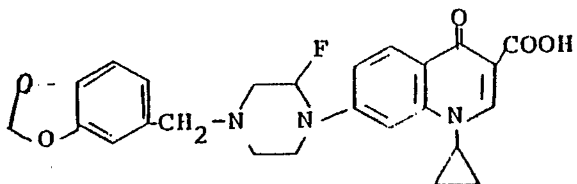 | 50 | 2 | 2 | 2 | 2 |
| 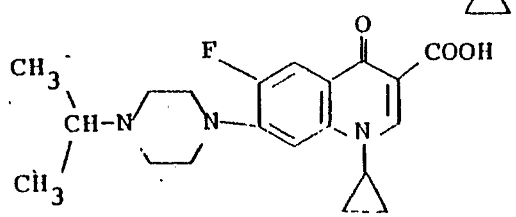 | 50 | 2 | 2 | 1 | 2 |

Beispiel D

Agarplatten-Test

Verwendeter Nährboden

15 Gewichtsteile Agar-Agar
10 Gewichtsteile Saccharose
8 Gewichtsteile Caseinhydrolysat
4 Gewichtsteile Hefeextrakt
2 Gewichtsteile Dikaliumhydrogenphosphat
0,3 Gewichtsteile Magnesiumphosphat

werden in 1 000 ml destilliertem Wasser gelöst und 15 Min. bei 121 °C autoklaviert.

Lösungsmittel : 10 Gewichtsteile Dimethylformamid
Mengenverhältnis von Lösungsmittel zu Nährboden : 0,2 : 99,8

Zur Herstellung einer zweckmäßigen Wirkstoffzubereitung vermischt man 1 Gewichtsteil Wirkstoff mit der angegebenen Menge Lösungsmittel.
Das Konzentrat wird im genannten Mengenverhältnis mit dem flüssigen Nährboden gründlich vermischt und in Petrischalen gegossen.
Ist der Nährboden erkaltet und fest, werden die Platten mit Mikroorganismen beimpft und bei ca. 28 °C inkubiert :
Die Auswertung erfolgt nach 2 Tagen, wobei die Wachstumshemmung als Maß für die Wirkung der Präparate herangezogen wird.
Eine deutliche Überlegenheit gegenüber dem Stand der Technik zeigen in diesem Test die Verbindungen gemäß folgender Herstellungsbeispiele :

(Siehe Tabelle D Seite 4 ff.)

Tabelle D

Agarplatten-Test

| Wirkstoffe | Wirkstoffkonzentration ppm | Wachstum in Wertzahlen (1 = kein Wachstum, 9 = Kontrolle) Mikroorganismen Xanthomonas oryzae | Xanthomonas vesicatoria | Pseudomonas lachrymans |
|---|---|---|---|---|
| 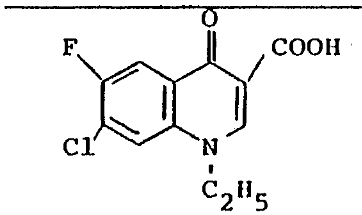 (bekannt) | 10 | 3 | 3 | 9 |
| 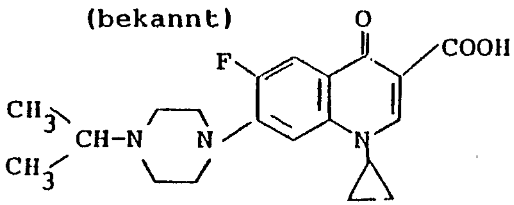 | 10 | 2 | 2 | 5 |
| 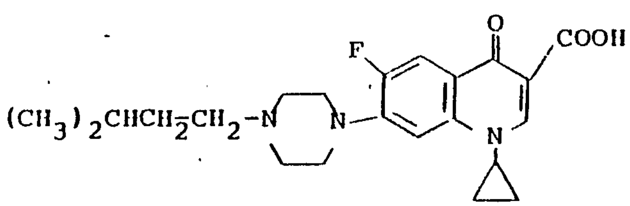 | 10 | 2 | 2 | 6 |

Tabelle D (Fortsetzung)

Agarplatten-Test

| Wirkstoffe | Wirkstoffkonzentration ppm | Wachstum in Wertzahlen (1 = kein Wachstum, 9 = Kontrolle) Mikroorganismen: Xanthomonas oryzae | Xanthomonas vesicatoria | Pseudomonas lachrymans |
|---|---|---|---|---|
| 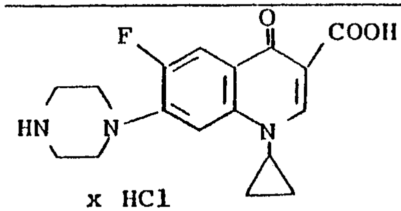 x HCl | 10 | 1 | 1 | 2 |
| 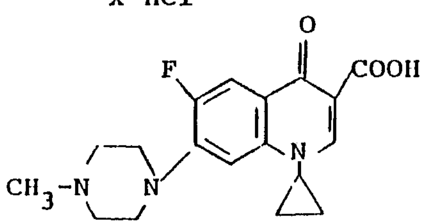 | 10 | 1 | 1 | 3 |
| 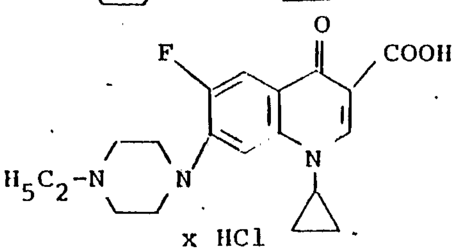 x HCl | 10 | 2 | 2 | 4 |

## Herstellungsbeispiele

### Beispiel 1

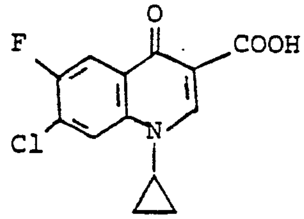

Eine Lösung von 31,9 g 2-(2,4-Dichlor-5-fluor-benzoyl)-3-cyclopropylamino-acrylsäure-ethylester in 300 ml wasserfreiem Dioxan wird unter Eiskühlung und Rühren portionsweise mit 3,44 g 80-proz. Natriumhydrid versetzt. Dann wird 30 Min. bei Raumtemperatur und 2 h unter Rückfluß gerührt und das Dioxan im Vakuum abgezogen. Der Rückstand (40,3 g) wird in 150 ml Wasser suspendiert, mit 6,65 g Ätzkali versetzt und 1,5 h refluxiert. Man filtriert die warme Lösung und wäscht mit Wasser nach. Dann wird mit halbkonz. Salzsäure unter Eiskühlung auf pH = 1-2 angesäuert, der Niederschlag abgesaugt, mit Wasser gewaschen und im Vakuum bei 100 °C getrocknet. Man erhält auf diese Weise 27,7 g 7-Chlor-1-cyclopropyl-6-fluor-1,4-dihydro-4-oxo-3-chinolincarbonsäure vom Schmelzpunkt 234-237 °C.

Herstellung des Ausgangsproduktes

24,3 g Magnesiumspäne werden in 50 ml wasserfreiem Ethanol suspendiert. Man versetzt mit 5 ml Tetrachlorkohlenstoff und tropft, wenn die Reaktion in Gang gekommen ist, ein Gemisch von 160 g Malonsäurediethylester, 100 ml abs. Ethanol und 400 ml wasserfreiem Ether zu, wobei ein heftiger Rückfluß zu beobachten ist. Nach dem Abklingen der Reaktion wird noch 2 h zum Sieden erhitzt, mit Trockeneis/Aceton auf — 5 °C bis — 10 °C gekühlt und bei dieser Temperatur eine Lösung von 227,5 g 2,4-Dichlor-5-fluor-benzoyl-chlorid in 100 ml abs. Ether langsam zugetropft. Man rührt 1 h bei 0 °C bis — 5 °C, läßt über Nacht auf Raumtemperatur kommen und läßt unter Eiskühlung ein Gemisch von 400 ml Eiswasser und 25 ml konz. Schwefelsäure zulaufen. Die Phasen werden getrennt und zweimal mit Ether nachextrahiert. Die vereinigten Etherlösungen werden mit gesättigter Natriumchlorid-Lösung gewaschen, mit Natriumsulfat getrocknet und das Lösungsmittel im Vakuum abgezogen. Man erhält 349,5 g 2,4-Dichlor-5-fluor-benzoyl-malonsäurediethylester als Rohprodukt.

Eine Emulsion von 34,9 g rohem 2,4-Dichlor-5-fluor-benzoyl-malonsäure-diethylester in 50 ml Wasser wird mit 0,15 g p-Toluolsulfonsäure versetzt. Man erhitzt unter gutem Rühren 3 h zum Sieden, extrahiert die erkaltete Emulsion mehrmals mit Methylenchlorid, wäscht die vereinigten Methylenchlorid-Lösungen einmal mit gesättigter Natriumchlorid-Lösung, trocknet mit Natriumsulfat und destilliert das Lösungsmittel in Vakuum ab. Die Fraktionierung des Rückstands im Feinvakuum liefert 21,8 g 2,4-Dichlor-5-fluor-benzoylessigsäureethylester vom Siedepunkt 127-142 °C/0,09 mbar.

Ein Gemisch von 21,1 g 2,4-Dichlor-5-fluor-benzoyl-essigsäureethylester, 16,65 g o-Ameisensäureethylester und 18,55 g Acetanhydrid wird 2 h auf 150 °C erhitzt. Dann werden im Wasserstrahlvakuum und zuletzt im Feinvakuum bei einer Badtemperatur von 120 °C die flüchtigen Bestandteile abdestilliert. Zurück bleiben 25,2 g roher 2-(2,4-Dichlor-5-benzoyl)-3-ethoxy-acrylsäureethylester. Er ist genügend rein für die weiteren Umsetzungen.

Eine Lösung von 24,9 g 2-(2,4-Dichlor-5-fluor-benzoyl)-3-ethoxy-acrylsäureethylester in 80 ml Ethanol wird unter Eiskühlung und Rühren tropfenweise mit 4,5 g Cyclopropylamin versetzt. Wenn die exotherme Reaktion abgeklungen ist, wird noch 1 h bei Raumtemperatur gerührt, das Lösungsmittel im Vakuum abgezogen und der Rückstand aus Cyclohexan/Petrolether umkristallisiert. Man erhält 22,9 g 2-(2,4-Dichlor-5-fluor-benzoyl)-3-cyclopropylamino-acrylsäureethylester vom Schmelzpunkt 89-90 °C.

Analog Beispiel 1 können hergestellt werden :

2. 7-Chlor-1-cyclopropyl-6-nitro-1,4-dihydro-4-oxo-3-chinolincarbonsäure vom Schmelzpunkt 265-275 °C (Zers.)
3. 1-Cyclopropyl-6,7-difluor-1,4-dihydro-4-oxo-3-chinolincarbonsäure vom Schmelzpunkt 290 °C.
4. 7-Chlor-1-cyclopropyl-1,4-dihydro-4-oxo-3-chinolincarbonsäure vom Schmelzpunkt 308 °C.
5. 6,7-Dichlor-1-cyclopropyl-1,4-dihydro-4-oxo-3-chinolincarbonsäure vom Schmelzpunkt 265 °C.

### Beispiel 6

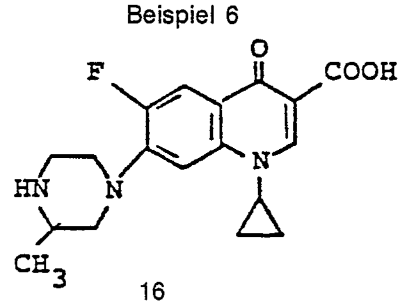

× 1/2 $H_2O$

Ein Gemisch aus 2,8 g (0,01 Mol) 7-Chlor-1-cyclopropyl-6-fluor-1,4-dihydro-4-oxo-3-chinolincarbonsäure und 5,1 g (0,051 Mol) 2-Methylpiperazin in 6 ml Dimethylsulfoxid wird 2 Stunden auf 140 °C erhitzt. Anschließend wird das Lösungsmittel im Hochvakuum abdestilliert, der Rückstand mit 6 ml heißem Wasser versetzt und 1 Stunde auf 95 °C gehalten. Man kühlt mit Eis, saugt den ausgefallenen Niederschlag ab, wäscht mit etwas Wasser und löst ihn in einer Mischung von 0,8 ml Essigsäure und 10 ml Wasser bei 90-100 °C auf. Das Filtrat wird mit Kalilauge (0,75 g KOH in 0,7 ml Wasser) auf pH 8 gebracht und der ausgefallene Niederschlag aus Methanol umkristallisiert. Man erhält 1,8 g (52 % d. Th.) 1-Cyclopropyl-6-fluor-1,4-dihydro-4-oxo-7-(3-methyl-1-piperazinyl)-3-chinolincarbonsäure-semihydrat mit einem Zersetzungspunkt von 230-232 °C.

Beispiel 7

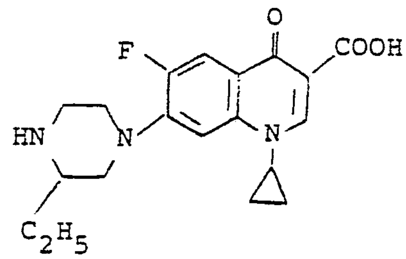

$\times H_2O$

Eine Mischung von 2,8 g (0,01 Mol) 7-Chlor-1-cyclopropyl-6-fluor-1,4-dihydro-4-oxo-3-chinolincarbonsäure und 3,4 g (0,03 Mol) 2-Ethylpiperazin in 15 ml Dimethylsulfoxid wird 2 Stunden auf 140 °C erhitzt. Die Lösung wird im Hochvakuum eingeengt, der Rückstand mit 30 ml Wasser auf 90 °C erhitzt, der ausgefallene Niederschlag abgesaugt, mit Wasser und Methanol gewaschen und isoliert. Man erhält 1,1 g (31 % der Theorie) 1-Cyclopropyl-6-fluor-1,4-dihydro-4-oxo-7-(3-ethyl-1-piperazinyl)-3-chinolincarbonsäure-monohydrat mit einem Zersetzungspunkt von 255-258 °C.

Beispiel 8

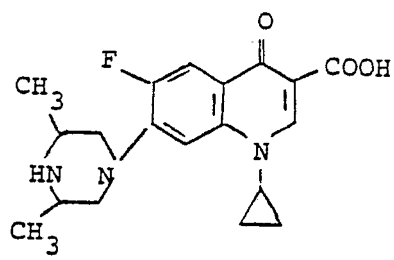

Ein Gemisch aus 2,8 g (0,01 Mol) 7-Chlor-1-cyclopropyl-6-fluor-1,4-dihydro-4-oxo-3-chinolincarbonsäure, 1,14 g (0,01 Mol) cis-2,6-Dimethylpiperazin und 2,2 g Diazabicyclo-[2,2,2] octan wird 5 Stunden auf 140 °C erhitzt. Das Lösungsmittel wird im Hochvakuum abdestilliert, der Rückstand mit 30 ml Wasser versetzt, die Suspension mit 2n-Salzsäure auf pH 8 eingestellt und der ausgefallene Niederschlag mit 30 ml Methanol ausgekocht. Man erhält 0,75 g (21 % der Theorie) 1-Cyclopropyl-6-fluor-1,4-dihydro-4-oxo-7-(3,5-dimethyl-1-piperazinyl)-3-chinolincarbonsäure vom Zersetzungspunkt 234-236 °C.

Massenspektrum : 359 ($M^+$), 290, 289 (100 %, $M^+$- 70), 245, 70

Beispiel 9

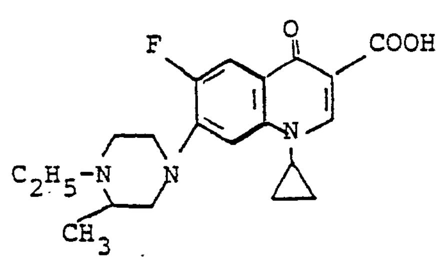

$\times HI \times H_2O$

Ein Gemisch aus 1,7 g (0,005 Mol) 1-Cyclopropyl-6-fluor-1,4-dihydro-4-oxo-7-(3-methyl-1-piperazinyl)-3-chinolincarbonsäure, 1,7 g (0,011 Mol) Ethyliodid und 1,1 g Triethylamin in 20 ml Dimethylformamid wird 3 Stunden auf 80 °C erhitzt. Die Lösung wird im Vakuum eingeengt, der kristalline Rückstand mit 10

ml Wasser verrührt und das ungelöste Produkt aus Methanol umkristallisiert. Man erhält 0,6 g (32 % der Theorie) 1-Cyclopropyl-6-fluor-1,4-dihydro-4-oxo-7-(4-ethyl-3-methyl-1-piperazinyl)-3-chinolincarbonsäure-hydroiodid-monohydrat mit einem Zersetzungspunkt von 285-288 °C.

Beispiel 10

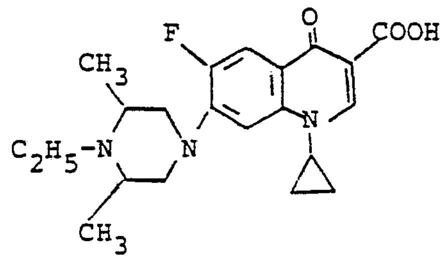

× HI

Man setzt 1,8 g (0,005 Mol) 1-Cyclopropyl-6-fluor-1,4-dihydro-4-oxo-7-(3,5-dimethyl-1-piperazinyl)-3-chinolincarbonsäure analog Beispiel 9 um und isoliert 0,75 g (39 %) 1-Cyclopropyl-6-fluor-1,4-dihydro-4-oxo-7-(4-ethyl-3,5-dimethyl-1-piperazinyl)-3-chinolincarbonsäure-hydroiodid mit einem Zersetzungspunkt von 277-279 °C.

Analog Beispiel 9 werden folgende Verbindungen erhalten :

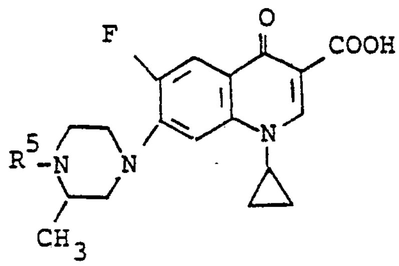

| Beispiel | $R^5$ | Schmelzpunkt /°C/ |
|---|---|---|
| 11 | $n-C_3H_7$ x HBr | 293-295 (Zersetzung) |
| 12 | $i-C_3H_7$ x HI | 289-291 " |
| 13 | $i-C_4H_9$ | 198-200 |
| 14 | $n-C_5H_9$ x HCl | 238-240 (Zersetzung) |
| 15 | $n-C_{12}H_{25}$ x HCl | 142-145 |
| 16 | $HO-CH_2-CH_2-CH_2-$ | 198-201 (Zersetzung) |
| 17 | $HO-CH_2-CH_2-$ x HCl | 240-243 " |

Beispiel 18

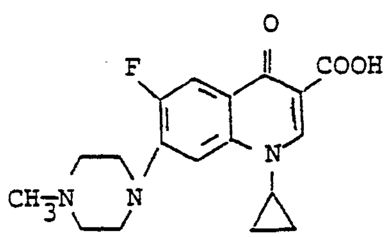

Ein Gemisch von 20 g 7-Chlor-1-cyclopropyl-6-fluor-1,4-dihydro-4-oxo-3-chinolincarbonsäure, 28,5 g N-Methylpiperazin und 120 ml wasserfreiem Dimethylsulfoxid wird 2,5 h auf 135-140 °C erhitzt. Das Lösungsmittel wird im Feinvakuum abdestilliert und der Rückstand in ca. 50 ml Wasser suspendiert. Man saugt ab, wäscht mit Wasser nach, trocknet im Vakuumtrockenschrank bei 80 °C über Calciumchlorid und kristallisiert aus Glykolmonomethylether um. Es werden 14,5 g 1-Cyclopropyl-6-fluor-1,4-dihydro-4-oxo-7-(4-methyl-1-piperazinyl)-3-chinolincarbonsäure vom Zersp. 248-250 °C erhalten.

Beispiel 19

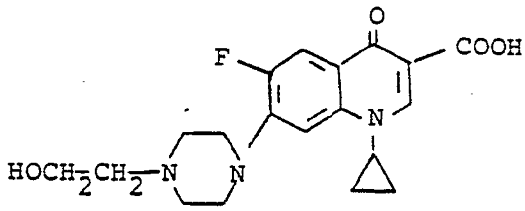

Eine Suspension von 2,81 g 7-Chlor-1-cyclopropyl-6-fluor-1,4-dihydro-4-oxo-3-chinolincarbonsäure und 5,2 g N-(2-hydroxyethyl)-piperazin in 25 ml Dimethylsulfoxid wird 2 h auf 135-140 °C erhitzt. Das Lösungsmittel wird im Feinvakuum abdestilliert, der Rückstand mit 20 ml Wasser kurz aufgekocht, über Nacht bei Raumtemperatur stehen gelassen, der Niederschlag unter Eiskühlung abgesaugt, mit Wasser gewaschen und im Vakuum über Calciumchlorid bei 80 °C getrocknet. Man erhält 2,1 g 1-Cyclopropyl-6-fluor-1,4-dihydro-4-oxo-7-[4-(2-hydroxyethyl)-1-piperazinyl]-3-chinolincarbonsäure vom Zersp. 237-239 °C. Massenspektrum m/e : 375 ($M^+$).

Beispiel 20

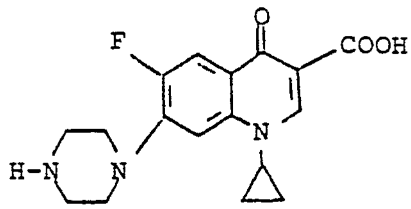

Ein Gemisch von 19,7 g 7-Chlor-1-cyclopropyl-6-fluor-1,4-dihydro-4-oxo-3-chinolincarbonsäure, 30,1 g wasserfreiem Piperazin und 100 ml Dimethylsulfoxid wird 2 h auf 135-140 °C erhitzt. Das Lösungsmittel wird im Feinvakuum abdestilliert, der Rückstand in Wasser suspendiert, abgesaugt und mit Wasser gewaschen. Zur weiteren Reinigung wird das feuchte Rohprodukt mit 100 ml Wasser aufgekocht, bei Raumtemperatur abgesaugt, mit Wasser gewaschen und im Vakuumtrockenschrank über Calciumchlorid bei 100 °C bis zur Gewichtskonstanz getrocknet. Man erhält 19,6 g 1-Cyclopropyl-6-fluor-1,4-dihydro-4-oxo-7-(1-piperazinyl)-3-chinolincarbonsäure vom Zersp. 255-257 °C.

Beispiel 21

1-Cyclopropyl-6-fluor-1,4-dihydro-4-oxo-7-(1-piperazinyl)-3-chinolincarbonsäure-hydrochlorid.

Die nach obigem Beispiel hergestellte Verbindung wird in 50 ml 10-proz. Salzsäure heiß gelöst. Zur filtrierten Lösung gibt man 150 ml Ethanol, kühlt mit Eis, saugt ab und wäscht mit Alkohol und trocknet im Vakuum bei 100 °C. Es werden 18,5 g 1-Cyclopropyl-6-fluor-1,4-dihydro-4-oxo-7-(1-piperazinyl)-3-chinolincarbonsäure-hydrochlorid als farblose Kristalle vom Zersp. 308-310 °C erhalten.

Beispiel 22

Natriumsalz der 1-Cyclopropyl-6-fluor-1,4-dihydro-4-oxo-7-(1-piperazinyl)-3-chinolincarbonsäure 68 g gepulverte 1-Cyclopropyl-6-fluor-1,4-dihydro-4-oxo-7-(1-piperazinyl)-3-chinolincarbonsäure werden in 300 ml Wasser suspendiert. Man versetzt mit einer Lösung von 7,7 g Ätznatron in 30 ml Wasser und rührt 30 Min. bei Raumtemperatur, wobei die Carbonsäure zum größten Teil in Lösung geht. Vom ungelösten Produkt wird abfiltriert, mit Wasser nachgespült und das Filtrat im Vakuum eingeengt. Der gelbliche kristalline Rückstand wird im Vakuum bei 100 °C getrocknet. Ausbeute 73,4 g Natriumsalz. Schmp. > 325 °C.

Entsprechend den Beispielen 18-20 lassen sich die folgenden Verbindungen der allgemeinen Formel I herstellen :

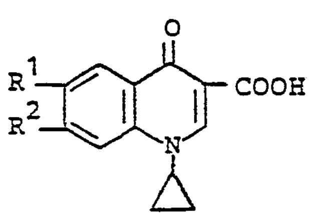

(I)

| Beispiel Nr. | $R^1$ | $R^2$ | Schmp. (°C) |
|---|---|---|---|
| 23 | F | 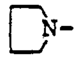 | 323 (Z) *) |
| 24 | H | 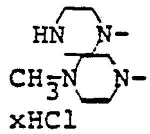 | 300 (Z) |
| 25 | H | xHCl | 320 (Z) |
| 26 | H | 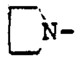 | >325 |
| 27 | F | 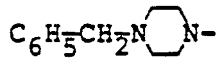 | 240 |
| 28 | F | 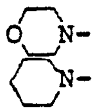 | 290 (Z) |
| 29 | F |  | 248 |
| 30 | F | 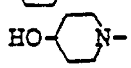 | 239 |
| 31 | F | 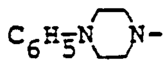 | 251 |
| 32 | F | 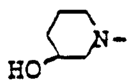 | 254 |
| 33 | Cl | 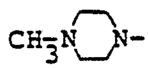 | 270 (Z.) |
| 34 | $NO_2$ | 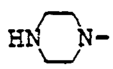 | 304 (Z.) |
| 35 | $NO_2$ | 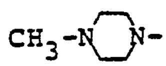 | 260 (Z.) |
| 36 | $NO_2$ | 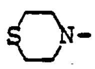 | 231 |
| 37 | F | 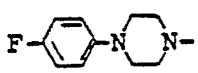 | 258 |
| 38 | F | 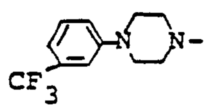 | 266 |
| 39 | F | 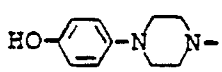 | 269 (Z.) |
| 40 | F | 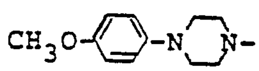 | 259 |
| 41 | F | 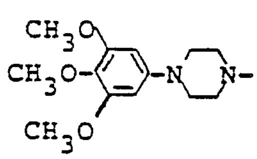 | 258 (Z.) |
| 42 | F | 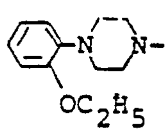 | 189 |

(Fortsetzung)

| Beispiel Nr. | $R^1$ | $R^2$ | Schmp. (°C) |
|---|---|---|---|
| 43 | F | 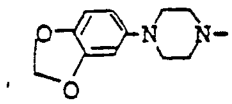 | 270 (Z.) |
| 44 | F | 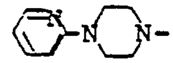 | 243 |
| 45 | F | 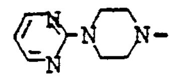 | 295 |
| 46 | F | 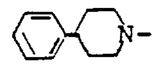 | 207 |
| 47 | F | 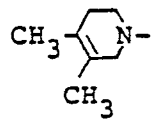 | 222 |
| 48 | F | 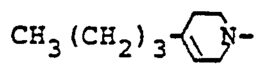 | 200 |
| 49 | F | 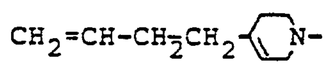 | 162 |
| 50 | F | 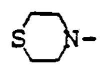 | 256 |
| 51 | F | 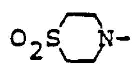 | 232 |
| 52 | F | 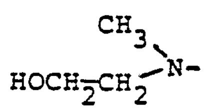 | 248 |
| 53 | F | 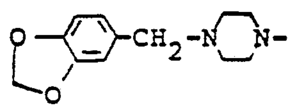 | 232 |
| 54 | F | 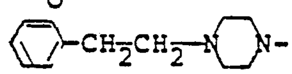 | 215 |
| 55 | F | 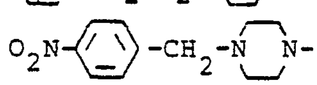 x HBr | 257 (Z.) |

*) Zersetzung

Beispiel 56

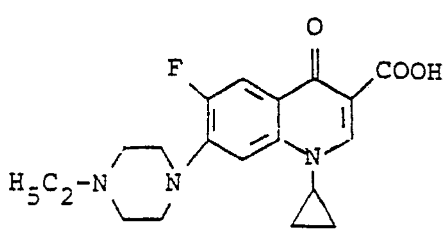

× HI

Ein Gemisch von 1,2 g 1-Cyclopropyl-6-fluor-1,4-dihydro-4-oxo-7-(1-piperazinyl)-3-chinolincarbonsäure, 1,13 g Ethyliodid, 0,73 g Triethylamin und 20 ml N,N-Dimethylformamid wird 2,5 h auf 70-80 °C erhitzt. Das Lösungsmittel wird im Vakuum abdestilliert und der Rückstand in Wasser suspendiert. Man saugt ab, wäscht mit Wasser nach und drückt auf Ton ab. Es werden 1,15 g 1-Cyclopropyl-6-fluor-7-(4-ethyl-1-piperazinyl)-1,4-dihydro-4-oxo-3-chinolincarbonsäure-hydroiodid vom Zersp. 306 °C erhalten. Massenspektrum m/e : 359 ($M^+$).

Beispiel 57

Betain

4,9 g der Verbindung aus Beispiel 56 werden in 35 ml Wasser suspendiert. Man versetzt mit einer Lösung von 0,86 g Natriumbicarbonat in 10 ml Wasser und rührt 1 h bei Raumtemperatur. Dann wird abgesaugt, mit 100 ml Wasser gewaschen und im Vakuum bei 100 °C getrocknet. Ausbeute : 4,4 g Betain vom Schmelzpunkt 205 °C.

Beispiel 58

Hydrochlorid von Beispiel 56

4,4 g Betain werden mit 10 ml halbkonz. Salzsäure kurz auf 100 °C erhitzt. Zur noch warmen Suspension des Hydrochlorids gibt man 30 ml Ethanol, kühlt mit Eis, saugt ab, wäscht gut mit Ethanol nach und trocknet im Vakuum bei 100 °C.

Ausbeute : 4,5 g Hydrochlorid vom Zersp. 328 °C.

Entsprechend Beispiel 55 können die folgenden Verbindungen der allgemeinen Formel I hergestellt werden :

| Beispiel Nr. | $R^1$ | $R^2$ | Schmp. (°C) |
|---|---|---|---|
| 59 | F | 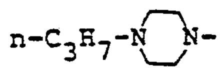 | 206 |
| 60 | F | 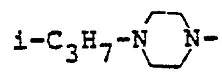 a)<br>b) x HI | 222-226 (Z.)<br>291 (Z.) |
| 61 | F | 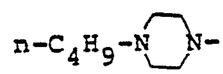 x HBr | 182 |
| 62 | F | 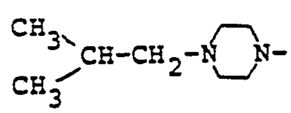 x HCl | 244 (Z.) |
| 63 | F | 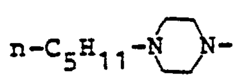 | 179 |
| 64 | F | 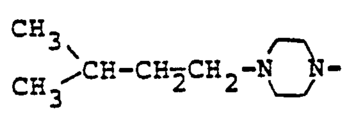 x HCl | 201 (Z.) |
| 65 | F | 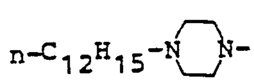 x HCl | 163 |
| 66 | F | 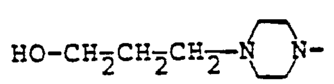 x HI | 291 (Z.) |
| 67 | F | 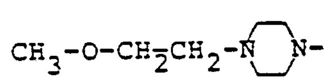 x. HCl | 250 (Z.) |

Beispiel 68

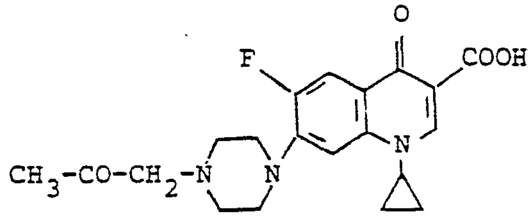

Eine Mischung von 23,2 g (0,07 Mol) 1-Cyclopropyl-6-fluor-1,4-dihydro-4-oxo-7-(1-piperazinyl)-3-chinolincarbonsäure mit 9,8 g Chloraceton wird in 350 ml Dimethylformamid mit 14,7 g Triethylamin 3 Stunden auf 80 °C erhitzt. Man engt im Hochvakuum ein, verrührt den Rückstand mit 140 ml Wasser und kristallisiert den ungelösten Feststoff aus Glykolmonomethylether um. Ausbeute : 17 g (62,8 % der Theorie) 1-Cyclopropyl-6-fluor-1,4-dihydro-4-oxo-7-[4-(2-oxopropyl)-1-piperazinyl]-3-chinolincarbonsäure mit einem Zersetzungspunkt von 220-225 °C.

Analog Beispiel 68 werden die folgenden Verbindungen hergestellt :

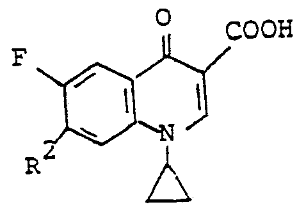

| Beispiel | $R^2$ | Schmelzpunkt [°C] |
|---|---|---|
| 69 | 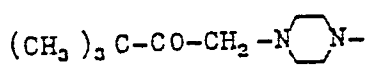 | 207-210 |
| 70 | 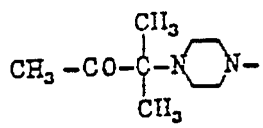 | 268-271 |
| 71 | 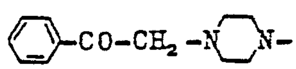 | 198-202 |
| 72 | 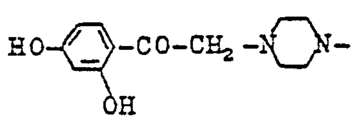 | 150-154 |
| 73 | 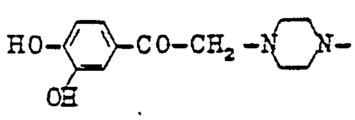 | 210-215 |
| 74 | 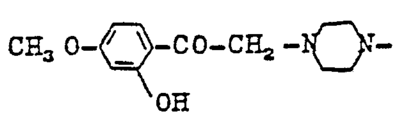 | 224-227 |
| 75 | 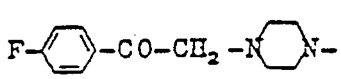 | 168-171 (Z.) |
| 76 | 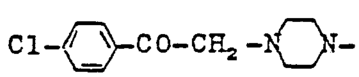 | 197-199 (Z.) |

Beispiel 77

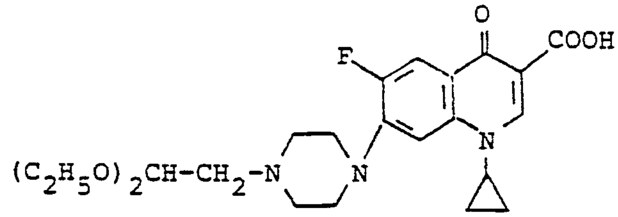

Eine Mischung aus 3,3 g (0,01 Mol) 1-Cyclopropyl-6-fluor-1,4-dihydro-4-oxo-7-(1-piperazinyl)-3-chinolincarbonsäure, 3,85 g (0,02 Mol) Bromacetaldehyddiethylacetal, 2,1 g Triethylamin und 3,35 g Kaliumiodid wird 11 Stunden auf 90 °C erhitzt. Die Lösung wird im Hochvakuum eingeengt, mit 20 ml Methanol verrührt und der ausgefallene Niederschlag mehrmals mit Wasser gewaschen und mit Methanol ausgekocht. Ausbeute : 1,3 g (29 %) 1-Cyclopropyl-7-[4-(2,2-diethoxyethyl)-1-piperazinyl]-6-fluor-1,4-dihydro-4-oxo-3-chinolincarbonsäure mit einem Zersetzungspunkt von 208-212 °C.

Beispiel 78

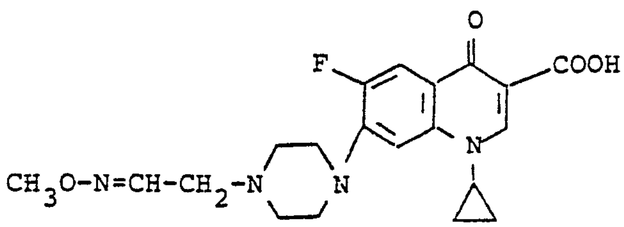 × HCl

Eine Mischung aus 3,3 g (0,01 Mol) 1-Cyclopropyl-6-fluor-1,4-dihydro-4-oxo-7-(1-piperazinyl)-3-chinolincarbonsäure, 1,6 g Chloracetaldehydoxim-O-methylether und 2,1 g Triethylamin wird 3 Stunden bei 80 °C gerührt. Anschließend wird im Hochvakuum eingeengt, mit 30 ml Wasser versetzt und mit 2n HCl auf pH 2 gestellt. Den Niederschlag saugt man ab, wäscht mit Wasser und Methanol und trocknet im Hochvakuum. Ausbeute : 1,9 g (43 % der Theorie) 1-Cyclopropyl-6-fluor-1,4-dihydro-7-[4-(2-methoximinoethyl)-1-piperazinyl]-4-oxo-3-chinolincarbonsäure-hydrochlorid mit einem Zersetzungspunkt von 215-221 °C.
NMR ($d_6$-DMSO) : δ 3,87 und 3,88 (2 Singuletts für die $CH_3O$-Gruppen der syn- und anti-Form).
Massenspektrum : m/e 402, 371, 331, 289, 287, 245 (100 %), 229, 70, 56, 44, 32, 31, 27.

Beispiel 79

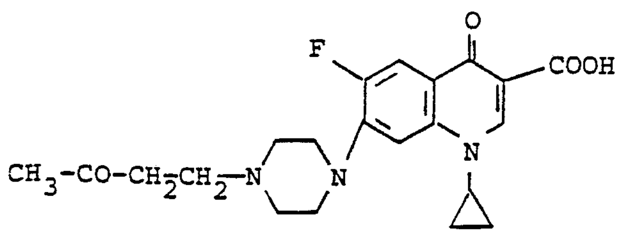

3,31 g (0,01 Mol) 1-Cyclopropyl-6-fluor-1,4-dihydro-4-oxo-7-(1-piperazinyl)-3-chinolincarbonsäure und 3,9 g Methylvinyl-keton werden in 50 ml Ethanol 2 Stunden unter Rückfluß erhitzt. Man saugt ab, wäscht mit Methanol und erhält 2,5 g (62,3 % der Theorie) 1-Cyclopropyl-6-fluor-1,4-dihydro-4-oxo-7-[4-(3-oxobutyl)-1-piperazinyl]-3-chinolincarbonsäure mit einem Zersetzungspunkt von 185-187 °C.

Beispiel 80

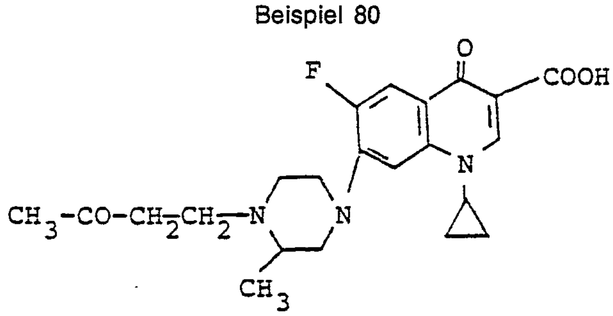

Analog Beispiel 79 erhält man mit dem Ausgangsprodukt aus Beispiel 6 1-Cyclopropyl-6-fluor-1,4-dihydro-7-[3-methyl-4-(3-oxobutyl)-1-piperazinyl]-4-oxo-3-chinolincarbonsäure (87 % der Theorie) mit einem Zersetzungspunkt von 176-178 °C.

Beispiel 81

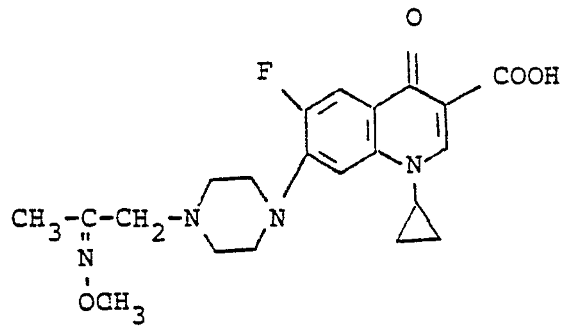

Eine Mischung aus 3,87 g (0,01 Mol) 1-Cyclopropyl-6-fluor-1,4-dihydro-4-oxo-7-[4-(2-oxopropyl)-1-piperazinyl]-3-chinolincarbonsäure und 835 mg (0,01 Mol) Methoxyaminhydrochlorid in 120 ml Ethanol wird mit 0,5 ml konzentrierter Salzsäure versetzt und 3 Stunden unter Rückfluß erhitzt. Die heiße Lösung wird mit etwas « Tonsil » (Bleicherde) verrührt und filtriert. Das nach dem Abkühlen ausfallende Kristallisat wird abgesaugt, mit Ether gewaschen und getrocknet. Ausbeute : 2,1 g (46 % der Theorie) 1-Cyclopropyl-6-fluor-1,4-dihydro-7-[4-(2-methoximinopropyl)-1-piperazinyl]-4-oxo-3-chinolincarbonsäure mit einem Zersetzungspunkt von 215-217 °C.

Analog Beispiel 81 werden die folgenden Verbindungen erhalten :

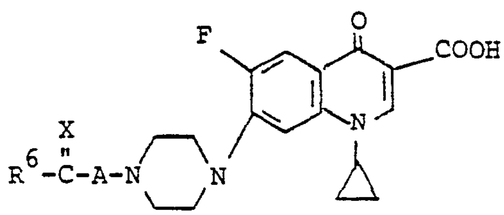

| Beispiel | 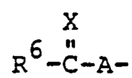 | Zersetzungspunkt |
|---|---|---|
| 82 | 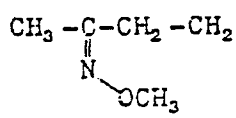 x HCl | 315-320° C (Zers.) |
| 83 | 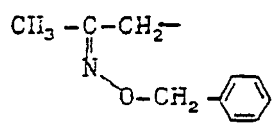 | 184-188° C (Zers.) |
| 84 | 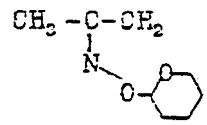 | 105-110° C (Zers.)+) |
| 85 | 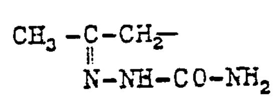 x HCl x $2H_2O$ | 217-219° C (Zers.) |
| 86 | 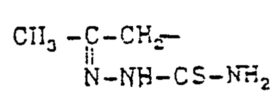 x HCl X $H_2O$ | 219-221° C (Zers.) |

+) erstarrter Schaum ; zum Teil ist der Tetrahydropyranylrest abgespalten

Beispiel 87

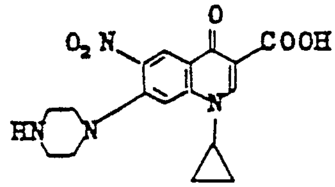

Eine Mischung von 9,3 g (0,03 Mol) 7-Chlor-1-cyclopropyl-1,4-dihydro-6-nitro-4-oxo-3-chinolincarbonsäure und 12,9 g (0,15 Mol) Piperazin wird in 60 ml Dimethylsulfoxid 15 Minuten auf 120 °C erwärmt. Aus der heißen Lösung fällt nach kurzer Zeit ein Niederschlag aus. Man engt im Hochvakuum ein, verrührt mit 30 ml Wasser und erhitzt noch 30 Minuten auf 95 °C. Mit 2n-Salzsäure wird die Mischung auf pH 8 eingestellt, der Niederschlag abgesaugt und mit Wasser und Methanol gewaschen. Man isoliert 5,8 g (54 % d. Th.) 1-Cyclopropyl-1,4-dihydro-6-nitro-4-oxo-7-(1-piperazinyl)-3-chinolincarbonsäure mit einem Zersetzungspunkt von 296-298 °C.

Beispiel 88

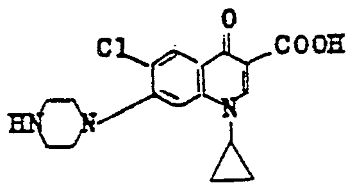

Man setzt analog Beispiel 87 6,7-Dichlor-1-cyclopropyl-1,4-dihydro-4-oxo-3-chinolincarbonsäure zu 1-Cyclopropyl-6-chlor-1,4-dihydro-4-oxo-7-(1-piperazinyl)-3-chinolincarbonsäure mit einem Zersetzungspunkt von 295-298 °C um.

Beispiel 89

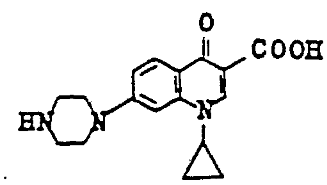

Man setzt analog Beispiel 87 7-Chlor-1-cyclopropyl-1,4-dihydro-4-oxo-3-chinolincarbonsäure mit Piperazin zu 1-Cyclopropyl-1,4-dihydro-4-oxo-7-(1-piperazinyl)-3-chinolincarbonsäure mit einem Zersetzungspunkt von 298-300 °C um.

**Patentansprüche**

1. Mikrobizide Verwendung von 1-Cyclopropyl-1,4-dihydro-4-oxo-chinolincarbonsäuren der Formel

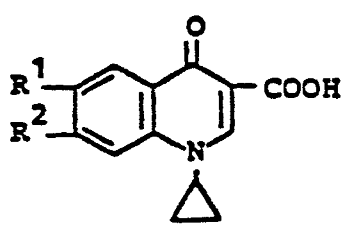

(I)

in welcher

$R^1$ für Wasserstoff, Fluor, Chlor, Brom oder Nitro steht,

$R^2$ für Wasserstoff, Chlor, Fluor oder für die Gruppe

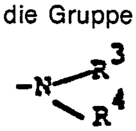

steht, wobei

$R^3$ und $R^4$ gleich oder verschieden sind und für gegebenenfalls durch Hydroxy substituiertes Alkyl

mit 1 bis 4 Kohlenstoffatomen stehen oder $R^3$ und $R^4$ gemeinsam mit dem Stickstoffatom, an dem sie stehen, einen 5- oder 6-gliedrigen, gesättigten oder partiell ungesättigten, gegebenenfalls ein- oder mehrfach, gleich oder verschieden substituierten Heterocyclus bilden, der gegebenenfalls noch Sauerstoff, Schwefel, eine SO-, $SO_2$- oder $NR^5$-Gruppe enthalten kann, wobei als Substituenten der Heterocyclen Alkyl mit 1 bis 6 Kohlenstoffatomen, Alkenyl mit 2 bis 6 Kohlenstoffatomen, Phenyl oder Hydroxy in Betracht kommen, und

$R^5$ für Wasserstoff, für gegebenenfalls ein- oder mehrfach, gleich oder verschieden durch Hydroxy oder Alkoxy mit 1 bis 4 Kohlenstoffatomen substituiertes Alkyl mit 1 bis 12 Kohlenstoffatomen, für gegebenenfalls durch Nitro, Amino oder die Gruppe $—O—CH_2—O—$ substituiertes Phenylalkyl mit 1 bis 3 Kohlenstoffatomen im Alkylteil, für gegebenenfalls ein- oder mehrfach, gleich oder verschieden durch Halogen, Halogenalkyl mit 1 oder 2 Kohlenstoffatomen und mit bis zu 5 Halogenatomen, Hydroxy, Alkoxy mit 1 bis 3 Kohlenstoffatomen oder durch die Gruppe $—O—CH_2—O—$ substituiertes Phenyl, für Heteroaryl oder für die Gruppierung

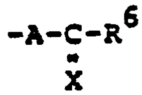

steht, wobei

A für eine Alkylenkette mit 1 bis 4 Kohlenstoffatomen steht,

$R^6$ für Wasserstoff, für Alkyl mit 1 bis 6 Kohlenstoffatomen oder für gegebenenfalls ein- oder mehrfach durch Hydroxy, Alkoxy oder Halogen substituiertes Phenyl steht und

X für Sauerstoff oder die Gruppierungen = NOR', = N—NH—R'' oder $(OR''')_2$ steht, wobei

R' für Wasserstoff, Alkyl mit 1 bis 6 Kohlenstoffatomen, Cycloalkyl mit 5 oder 6 Kohlenstoffatomen, Benzyl, Chlorbenzyl oder Tetrahydropyranyl steht,

R'' für Methyl, Phenyl, Carbamoyl oder Thiocarbamoyl und

R''' für Methyl, Ethyl oder $(OR''')_2$ für

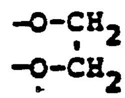

stehen, und deren pflanzenverträgliche Säureadditions-, Alkali-, Erdalkali- oder Schwermetallsalze und deren Hydrate zur Bekämpfung von Mikroorganismen im Pflanzenschutz.

2. Mikrobizide Verwendung von 1-Cyclopropyl-1,4-dihydro-4-oxo-chinolincarbonsäuren der Formel (I) gemäß Anspruch 1, in welcher

$R^1$ für Wasserstoff, Fluor, Chlor oder für Nitro steht,

$R^2$ für Chlor, Fluor oder für die Gruppe

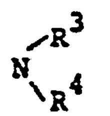 ,

wobei

$R^3$ und $R^4$ gleich oder verschieden sind und für Alkyl mit 1 bis 3 Kohlenstoffatomen, für einfach durch Hydroxy substituiertes Alkyl mit 2 oder 3 Kohlenstoffatomen stehen oder $R^3$ und $R^4$ gemeinsam mit dem Stickstoffatom, an dem sie stehen, für gegebenenfalls ein- bis dreifach durch Alkyl mit 1 bis 4 Kohlenstoffatomen, Alkenyl mit 2 bis 4 Kohlenstoffatomen, Phenyl oder durch Hydroxy substituiertes 1-Pyrrolidinyl, 1-Piperidinyl, 1,2,3,6-Tetrahydro-1-pyridyl, 4-Morpholinyl, 4-Thiomorpholinyl, 1,1-Dioxo-4-thiomorpholinyl stehen oder $R^3$ und $R^4$ gemeinsam mit dem Stickstoffatom, an dem sie stehen, den Heterocyclus

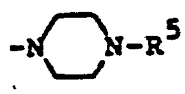

bilden, der wie angegeben substituiert sein kann und

$R^5$ für Wasserstoff, für Alkyl mit 1 bis 12 Kohlenstoffatomen, für einfach durch Hydroxy substituiertes Alkyl mit 1 bis 4 Kohlenstoffatomen, für einfach durch Methoxy substituiertes Alkyl mit 1 bis 4 Kohlenstoffatomen, für gegebenenfalls ein- bis dreifach durch Hydroxy, Alkoxy mit 1 oder 2 Kohlenstoffatomen, Trifluormethyl, Fluor, Dioxymethylen substituiertes Phenyl, für Pyridyl, für Pyrimidinyl, für gegebenenfalls durch Dioxymethylen substituiertes Phenalkyl mit 1 oder 2 Kohlenstoffatomen im Alkylteil oder für die Gruppierung

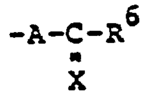

steht, wobei

A für eine Alkylenkette mit 1 bis 3 Kohlenstoffatomen steht,

$R^6$ für Wasserstoff, für Alkyl mit 1 bis 4 Kohlenstoffatomen, für gegebenenfalls ein- bis dreifach, gleich oder verschieden durch Hydroxy, Fluor, Chlor oder Methoxy substituiertes Phenyl und

X für Sauerstoff oder die Gruppierungen = NOR′, = N—NHR″ oder $(OR''')_2$ stehen, wobei

R′ für Alkyl mit 1 bis 3 Kohlenstoffatomen, für Benzyl oder für Tetrahydropyranyl steht,

R″ für Methyl, Ethyl, Carbamoyl oder Thiocarbamoyl und

R‴ für Methyl oder $(OR''')_2$ für

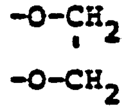

stehen und deren pflanzenverträgliche Säureadditions-, Alkali-, Erdalkali- oder Schwermetallsalze und die Hydrate zur Bekämpfung von Mikroorganismen im Pflanzenschutz.

3. Verfahren zur Bekämpfung von Mikroorganismen im Pflanzenschutz, dadurch gekennzeichnet, daß man 1-Cyclopropyl-1,4-dihydro-4-oxo-chinolincarbonsäuren der Formel (I) gemäß Anspruch 1 oder deren pflanzenverträgliche Säureadditions-, Alkali-, Erdalkali- oder Schwermetallsalze oder Hydrate auf Mikroben oder ihren Lebensraum einwirken läßt.

**Claims**

1. Microbicidal use of 1-cyclopropyl-1,4-dihydro-4-oxo-quinolinecarboxylic acids of the formula (I)

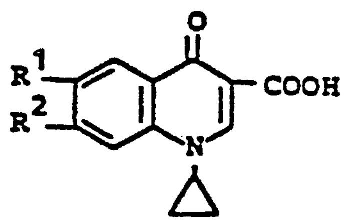

in which

$R^1$ represents hydrogen, fluorine, chlorine, bromine or nitro,

$R^2$ represents hydrogen, chlorine, fluorine or the group

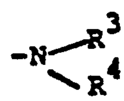

wherein

$R^3$ and $R^4$ are identical or different and represent optionally hydroxyl-substituted alkyl with 1 to 4 carbon atoms or $R^3$ and $R^4$, together with the nitrogen atom on which they are positioned, form a 5- or 6-membered, saturated or partially unsaturated heterocyclic radical which is optionally mono-or polysubstituted by identical or different substituents and can optionally additionally contain oxygen, sulphur or an SO, $SO_2$ or $NR^5$ group, possible substituents of the heterocyclic radicals being alkyl with 1 to 6 carbon atoms, alkenyl with 2 to 6 carbon atoms, phenyl or hydroxyl, and

$R^5$ represents hydrogen, alkyl which has 1 to 12 carbon atoms and is optionally mono- or polysubstituted by identical or different substituents from the group comprising hydroxyl and alkoxy with 1 to 4 carbon atoms, phenylalkyl which has 1 to 3 carbon atoms in the alkyl part and is optionally substituted by nitro, amino or the group $—O—CH_2—O—$, phenyl which is optionally mono- or polysubstituted by identical or different substituents from the group comprising halogen, halogenoalkyl with 1 or 2 carbon atoms and with up to 5 halogen atoms, hydroxyl, alkoxy with 1 to 3 carbon atoms and the group $—O—CH_2—O—$, heteroaryl or the grouping

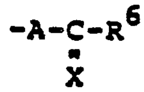

wherein

A represents an alkylene chain with 1 to 4 carbon atoms,

$R^6$ represents hydrogen, alkyl with 1 to 6 carbon atoms or phenyl which is optionally mono- or polysubstituted by hydroxyl, alkoxy or halogen and

X represents oxygen or the groupings = NOR′, = N—NH—R″ or (OR‴), wherein

R′ represents hydrogen, alkyl with 1 to 6 carbon atoms, cycloalkyl with 5 or 6 carbon atoms, benzyl, chlorobenzyl or tetrahydropyranyl,

R″ represents methyl, phenyl, carbamoyl or thiocarbamoyl and
R‴ represents methyl or ethyl or $(OR''')_2$ represents

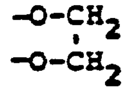

and their plant-tolerated acid addition, alkali metal, alkaline earth metal or heavy metal salts and their hydrates, for combating microorganisms in plant protection.

2. Microbicidal use of 1-cyclopropyl-1,4-dihydro-4-oxo-quinolinecarboxylic acids of the formula (I) according to Claim 1, in which

$R^1$ represents hydrogen, fluorine, chlorine or nitro,
$R^2$ represents chlorine, fluorine or the group

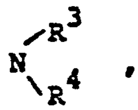 ,

wherein

$R^3$ and $R^4$ are identical or different and represent alkyl with 1 to 3 carbon atoms, alkyl which has 2 or 3 carbon atoms and is monosubstituted by hydroxyl or $R^3$ and $R^4$, together with the nitrogen atom on which they are positioned, represent 1-pyrrolidinyl, 1-piperidinyl, 1,2,3,6-tetrahydro-1-pyridyl, 4-morpholinyl, 4-thiomorpholinyl or 1,1-dioxo-4-thiomorpholinyl, each of which is optionally mono- to trisubstituted by alkyl with 1 to 4 carbon atoms, alkenyl with 2 to 4 carbon atoms, phenyl or hydroxyl or $R^3$ and $R^4$, together with the nitrogen atom on which they are positioned, form the heterocyclic radical

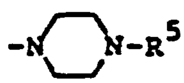

which can be substituted as mentioned and

$R^5$ represents hydrogen, alkyl with 1 to 12 carbon atoms, alkyl which has 1 to 4 carbon atoms and is monosubstituted by hydroxyl, alkyl which has 1 to 4 carbon atoms and is monosubstituted by methoxy, phenyl which is optionally mono- to trisubstituted by hydroxyl, alkoxy with 1 or 2 carbon atoms, trifluoromethyl, fluorine or dioxymethylene, pyridyl, pyrimidinyl, optionally dioxymethylene-substituted phenalkyl with 1 or 2 carbon atoms in the alkyl part or the grouping

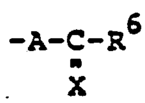

wherein

A represents an alkylene chain with 1 to 3 carbon atoms,

$R^6$ represents hydrogen, alkyl with 1 to 4 carbon atoms or phenyl which is optionally mono- to trisubstituted by identical or different substituents from the group comprising hydroxyl, fluorine, chlorine and methoxy, and

X represents oxygen or the groupings = NOR′, = N—NHR″ or $(OR''')_2$, wherein
R′ represent alkyl with 1 to 3 carbon atoms, benzyl or tetrahydropyranyl,
R″ represents methyl, ethyl, carbamoyl or thiocarbamoyl and
R‴ represents methyl or $(OR''')_2$ represents

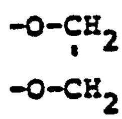

and their plant-tolerated acid addition, alkali metal, alkaline earth metal or heavy metal salts and the hydrates, for combating microorganisms in plant protection.

3. Process for combating microorganisms in plant protection, characterised in that 1-cyclopropyl-1,4-dihydro-4-oxo-quinolinecarboxylic acids of the formula (I) according to Claim 1 or their plant-tolerated acid addition, alkali metal, alkaline earth metal or heavy metal salts or hydrates are allowed to act on microbes or their environment.

**Revendications**

1. Utilisation microbicide d'acides 1-cyclopropyl-1,4-dihydro-4-oxo-quinoléine-carboxyliques de formule (I)

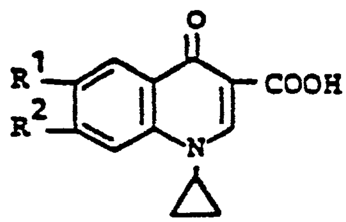

(I)

dans laquelle

$R^1$ représente de l'hydrogène, du fluor, du chlore, du brome ou un nitro,

$R^2$ de l'hydrogène, du chlore, du fluor ou le groupe

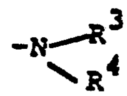

$R^3$ et $R^4$ sont identiques ou différents et représentent un alcoyle ayant 1 à 4 atomes de carbone éventuellement substitué par un hydroxy, ou bien $R^3$ et $R^4$, en commun avec l'atome d'azote auquel ils sont attachés, forment un hétérocycle à 5 ou 6 chaînons, saturé ou partiellement insaturé, éventuellement substitué une ou plusieurs fois, de manière identique ou différente, et qui le cas échéant peut contenir en plus de l'oxygène, du soufre, un groupe SO, $SO_2$ ou $NR^5$, étant envisagés comme substituants de l'hétérocycle un alcoyle ayant 1 à 6 atomes de carbone, un alcényle ayant 2 à 6 atomes de carbone, un phényle ou un hydroxy et

$R^5$ représente de l'hydrogène, un alcoyle ayant 1 à 12 atomes de carbone éventuellement substitué une ou plusieurs fois, de manière identique ou différente, par un hydroxy ou par un alcoxy ayant 1 à 4 atomes de carbone ; un phénylalcoyle ayant 1 à 3 atomes de carbone dans la portion alcoyle et éventuellement substitué par un nitro, un amino ou par le groupe $-O-CH_2-O-$ ; un phényle éventuellement substitué une ou plusieurs fois, de manière identique ou différente, par de l'halogène, par un halogénoalcoyle ayant 1 ou 2 atomes de carbone et avec jusqu'à 5 atomes d'halogène, par un hydroxy, par un alcoxy ayant 1 à 3 atomes de carbone ou par le groupe $-O-CH_2-O-$, un hétéroaryle ou le groupement

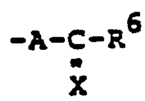

A représentant une chaîne alcoylène ayant 1 à 4 atomes de carbone,

$R^6$ de l'hydrogène, un alcoyle ayant 1 à 6 atomes de carbone ou un phényle éventuellement substitué une ou plusieurs fois par un hydroxy, un alcoxy, ou par de l'halogène, et

X représentant de l'oxygène ou les groupements = NOR', = N—NH—R'' ou $(OR''')_2$,

R' représentant de l'hydrogène, un alcoyle ayant 1 à 6 atomes de carbone, un cycloalcoyle ayant 5 ou 6 atomes de carbone, un benzyle, un chlorobenzyle ou un tétrahydropyranyle,

R'' un méthyle, phényle, carbamoyle ou thiocarbamoyle et

R''' un méthyle, éthyle ou $(OR''')_2$, un

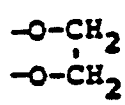

et de leurs sels d'addition d'acides, alcalins, alcalino-terreux ou de métaux lourds compatibles avec les plantes ainsi que de leurs hydrates, pour combattre les microorganismes dans la protection des plantes.

2. Utilisation microbicide d'acides 1-cyclopropyl-1,4-dihydro-4-oxo-quinoléine-carboxyliques de formule (I) selon la revendication 1, dans laquelle

$R^1$ représente de l'hydrogène, du fluor, du chlore ou un nitro,

$R^2$ représente du chlore, du fluor ou le groupe

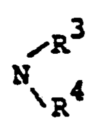 ,

$R^3$ et $R^4$ sont identiques ou différents et représentent un alcoyle ayant 1 à 3 atomes de carbone, un alcoyle ayant 2 ou 3 atomes de carbone substitué une fois par un hydroxy, ou bien $R^3$ et $R^4$, conjointement avec l'atome d'azote sur lequel ils sont fixés, représentent un 1-pyrrolidinyle, 1-pipérazinyle, 1,2,3,6-tétrahydro-1-pyridyle, 4-morpholinyle, 4-thiomorpholinyle, 1,1-dioxo-4-thiomorpholinyle éventuellement substitué une à trois fois par un alcoyle ayant 1 à 4 atomes de carbone, par un alcényle ayant 2 à 4 atomes de carbone, par un phényle, par un hydroxy ou encore $R^3$ et $R^4$, conjointement avec l'atome d'azote sur lequel ils sont fixés, forment l'hétérocycle

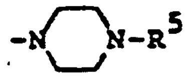

qui peut être substitué de la manière indiquée, et

$R^5$ représente de l'hydrogène, un alcoyle ayant 1 à 12 atomes de carbone, un alcoyle ayant 1 à 4 atomes de carbone et substitué une fois par un hydroxy, un alcoyle ayant 1 à 4 atomes de carbone et substitué une fois par un méthoxy, un phényl éventuellement substitué une à trois fois par un hydroxy, alcoxy ayant 1 ou 2 atomes de carbone, trifluorométhyle, fluor, dioxyméthylène ; un pyridyle ; un pyrimidyle ; un phénylalcoyle ayant 1 ou 2 atomes de carbone dans la portion alcoyle et éventuellement substitué par un dioxyméthylène ; ou le groupement

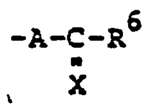

A étant une chaîne alcoylène ayant 1 à 3 atomes de carbone,

$R^6$ de l'hydrogène, un alcoyle ayant 1 à 4 atomes de carbone, un phényle éventuellement substitué une à trois fois, de manière identique ou différente, par un hydroxy, fluor, chlore ou méthoxy,

X de l'oxygène ou les groupements = NOR', = N—NHR'' ou $(OR''')_2$,

R' représentant un alcoyle ayant 1 à 3 atomes de carbone, un benzyle ou un tétrahydropyranyle,

R'' un méthyle, éthyle, carbamoyle ou thiocarbamoyle et

R''' un méthyle ou $(OR''')_2$, un

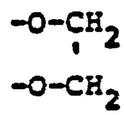

et de leurs sels d'addition d'acides; alcalins, alcalino-terreux ou de métaux lourds, compatibles avec les plantes, ainsi que de leurs hydrates, pour combattre les microorganismes dans la protection des plantes.

3. Procédé pour combattre les microorganismes dans la protection des plantes, caractérisé en ce qu'on fait agir des acides 1-cyclopropyl-1,4-dihydro-4-oxo-quinoléine-carboxyliques de formule (I) selon la revendication 1 ou leurs sels d'addition d'acides, alcalins, alcalino-terreux ou de métaux lourds compatibles avec les plantes, ou leurs hydrates, sur des microbes ou sur leur espace vital.